**Europäisches Patentamt**

**European Patent Office**

**Office européen des brevets**

(19)

(11) Veröffentlichungsnummer : **0 222 249**
B1

(12) # EUROPÄISCHE PATENTSCHRIFT

(45) Veröffentlichungstag der Patentschrift :
28.09.88

(51) Int. Cl.⁴ : **C 07 C121/56**, C 07 C120/14

(21) Anmeldenummer : **86114923.5**

(22) Anmeldetag : **27.10.86**

(54) Verfahren zur Herstellung von aromatischen Nitrilen.

(30) Priorität : **15.11.85 DE 3540517**

(43) Veröffentlichungstag der Anmeldung :
**20.05.87 Patentblatt 87/21**

(45) Bekanntmachung des Hinweises auf die Patenterteilung : **28.09.88 Patentblatt 88/39**

(84) Benannte Vertragsstaaten :
**BE DE FR GB IT NL**

(56) Entgegenhaltungen :
DE-B- 1 279 012
DE-B- 1 643 630
GB-A- 1 001 674
US-A- 3 945 804

(73) Patentinhaber : **BASF Aktiengesellschaft
Carl-Bosch-Strasse 38
D-6700 Ludwigshafen (DE)**

(72) Erfinder : **Engelbach, Heinz, Dr.
Kropsburgstrasse 24
D-6703 Limburgerhof (DE)**
Erfinder : **Krokoszinski, Roland, Dr.
Im Eiertal 4a
D-6719 Weisenheim (DE)**
Erfinder : **Franzischka, Wolfgang, Dr.
Franz-Lind-Strasse 1
D-6713 Freinsheim (DE)**
Erfinder : **Decker, Martin, Dr.
Maria-Stuart-Strasse 21
D-6700 Ludwigshafen (DE)**

EP 0 222 249 B1

## Beschreibung

Es ist bekannt, alkylsubstituierte aromatische Kohlenwasserstoffe mit Sauerstoff und Ammoniak in der Gasphase unter Verwendung von Katalysatoren umzusetzen. Für die Reaktion sind zahlreiche Katalysatoren bekannt, die Vanadiumoxid als aktive Katalysatorkomponente, neben anderen Metalloxiden, z. B. von Wolfram, Molybdän, Antimon, Wismut und Titan enthalten.

Die Selektivität und die Aktivität der bekannten Katalysatoren ist jedoch nicht zufriedenstellend. Außerdem kann man sie nur bis zu Kohlenwasserstoffkonzentrationen von nicht mehr als 2,0 Volumenprozent des Reaktionsgases verwenden.

Ein Nachteil der bekannten Verfahren besteht auch darin, daß sie die Herstellung von o-Phthalodinitril, welches für die Pigmentherstellung große technische Bedeutung hat, durch Ammonoxidation von o-Xylol praktisch nicht oder nur mit unbefriedigender Ausbeute ermöglichen.

Aus der DE-PS 1 643 630 ist ein Verfahren bekannt, welches durch Ammonoxidation der entsprechenden Kohlenwasserstoffe die aromatischen Mono- und Dinitrile verfügbar macht.

Die Erfindung geht aus von einem Verfahren zur Herstellung von aromatischen Nitrilen, insbesondere von Phthalsäuredinitrilen aus entsprechend alkylsubstituierten aromatischen Kohlenwasserstoffen durch katalytische Oxydation mit Sauerstoff oder Sauerstoff enthaltenden Gasen in Gegenwart von Ammoniak bei erhöhter Temperatur in der Dampfphase in Gegenwart eines Katalysators der aus einem 2 bis 10 Gew.% Vanadin(V)-oxid, 1 bis 10 Gew.% Antimon(III)-oxid, 0,02 bis 2 Gew.% Alkalioxid und Aluminiumoxid enthält.

Die Erfindung betrifft ein Verfahren dieser Art bei dem man die Selektivität der Reaktion und die Aktivität des Katalysators durch einen Zusatz von 0,01 bis 1,0 Gew.% eines Erdalkalimetalles, insbesondere durch Zusatz von 0,01 bis 1,0 % Barium zum Katalysator verbessert.

Es sind zwar aus DE-PS 1 279 012, US-PS 3 435 061 und GB-PS 1 065 444 schon Katalysatoren für die Ammonoxidation von aromatischen Kohlenwasserstoffen zu Nitrilen bekannt, die neben anderen aktiven Substanzen auch Barium enthalten können, jedoch zeigen die Beispiele, daß Barium in Verbindung mit diesen Stoffkombinationen eine beträchtlich niedrigere Selektivität des Katalysators ergibt als bariumfreie Varianten :

Erfindungsgemäß werden die Erdalkalimetalle, insbesondere Barium in einer Menge von 0,01 bis 1,0, vorzugsweise 0,2-0,7 Gew.% zugegeben. Durch diesen Zusatz wird überraschenderweise die Selektivität um etwa 5 Mol.% erhöht und die Totaloxidation zu Kohlendioxid sowie die Ammoniakverbrennung sind entsprechend abgeschwächt. Anstelle von Ba können auch äquivalente Mengen Sr, Ca oder Mg verwendet werden.

Neben der größeren Selektivität besteht ein weiterer Vorteil des erfindungsgemäßen Verfahrens darin, daß man die Volumenkonzentration des Kohlenwasserstoffes im Reaktionsgas noch erhöhen und den Partialdruck des Sauerstoffs erniedrigen kann, ohne daß es zur Ablagerung von Kohlenstoffverbindungen am Katalysator kommt, durch welche die Wirksamkeit des Katalysators schnell verloren geht. Mit dem Erfindungsgemäßen Katalysator kann der Durchsatz des aromatischen Kohlenwasserstoffs je Liter Reaktionsraum um ca. 20 % über den bisher möglichen Durchsatz erhöht und gleichzeitig das Molverhältnis Sauerstoff : Kohlenwasserstoff abgesenkt werden. Das verbesserte Verfahren erlaubt somit bei gegebener Produktionsanlage eine höhere Produktionsleistung und niedrigere Kosten.

Das für den Katalysator verwendete Aluminiumoxid erhält man, indem man ein Aluminiumhydroxid oder -oxidhydrat, vorzugsweise ein durch Fällen mit Ammoniak aus schwefelsaurer Lösung erhaltenes Aluminiumhydroxid in an sich bekannter Weise trocknet, mahlt, tablettiert und schließlich, beispielsweise 1/8 bis 2 Stunden, auf eine Temperatur zwischen 600 und 1 000 °C, vorzugsweise zwischen 800 und 900 °C, erhitzt. Das so hergestellte Aluminiumoxid stellt ein Gemisch verschiedener Modifikationen dar, in dem die $\gamma$-Modifikation vorherrscht.

Neben Aluminiumoxid enthält der Katalysator 2 bis 10 Gew.% Vanadium(V)-oxid, 1 bis 10 Gew.% Antimon(III)oxid, 0,02 bis 2 Gew.% Alkalioxide, vorteilhaft 3 bis 7 Gew.% Vanadium(V)-oxid, 3 bis 7 Gew.% Antimon(III)-oxid, 0,1 bis 1 Gew.% Alkalioxid und 0,1 bis 1,0 Gew.% eines Erdalkalimetalles oder einer Verbindung eines Erdalkalimetalles, insbesondere Barium oder einer Bariumverbindung. Als Alkalioxid können Oxide von Lithium, Natrium, Kalium, Rubidium oder Caesium oder Gemische derselben verwendet werden. Vorzugsweise setzt man Kaliumoxid ein. Die Prozentangaben beziehen sich auf die analytisch ermittelten Gehalte an den betreffenden Stoffen und bedeuten nicht, daß diese Stoffe als solche in den Katalysatoren vorliegen. Es ist vielmehr beispielsweise möglich, daß sie mit anderen Oxiden zu salzartigen Verbindungen reagiert haben.

Zur Herstellung der Katalysatoren können die genannten Metalloxide nach den üblichen Tränkmethoden auf den Aluminiumoxidträger aufgebracht werden, z. B. indem man ihn mit einer Lösung, die die Metallsalze in der gewünschten Menge enthält, tränkt und dann trocknet. Man kann auch ein feinvermahlenes Aluminiumoxidmehl mit der Metallsalzlösung zu einer Paste kneten und das voher getrocknete Gut zu der gewünschten Korngröße brechen. Antimon setzt man bevorzugt als Ammoniumantimonyltartrat oder als Antimonnitrat und die Erdalkalimetalle in Form ihrer löslichen Salze, z. B. der Nitrate ein, während Vanadium beispielsweise als Oxalat in der Metallsalzlösung vorliegen kann. Die Alkalimetalle setzt man zweckmäßig als Hydroxide ein. Anschließend überführt man die Metallsalze in die entsprechenden Oxide, z. B. indem man das getränkte Aluminiumoxid eine Zeitlang, z. B. 1 bis 20 Stunden, etwa auf 200 bis 600 °C, vorzugsweise auf 300 bis 450 °C in einem Strom aus Sauerstoff enthaltenden inerten Gasen erhitzt. Der Sauerstoffgehalt im Gasgemisch beträgt zweckmäßig 0,5 bis 20 Volumenprozent, vorteilhaft 2 bis 13 Volumenprozent. Als inerte Gase können beispielsweise Stickstoff, Kohlendioxid, Wasserdampf oder Gemisch derselben verwendet werden.

Besonders vorteilhaft erhält man die Katalysatoren jedoch, wenn man die Metallsalze in einer Operation auf den Träger aufbringt und zersetzt, z. B. indem man die Lösung der Metallsalze bei etwa 200 bis 450 °C, vorzugsweise bei 250 bis 350 °C, auf den Träger auftropft, der durch einen Sauerstoff enthaltenden Inertgasstrom, beispielsweise durch einen Stickstoff-Sauerstoff-Strom mit 0,5 bis 16 Volumenprozent, vornehmlich 8 bis 12 Volumenprozent Sauerstoff in wirbelnder Bewegung gehalten wird.

Für das Verfahren sind Oxidationsbedingungen geeignet, wie sie an sich bekannt sind.

Als Ausgangstoffe kann man ein- oder mehrfach, zweckmäßig bis zu 3-fach durch Alkylgruppen substituierte Benzole oder Naphthaline verwenden. Aus Toluol oder aus Ethylbenzol erhält man Benzonitril, aus den Xylolen die Phthalsäuredinitrile, Mesitylen liefert entsprechend Trimesinsäuretrinitril, Alkylnaphthaline liefern die entsprechenden Naphthalincarbonsäurenitrile ; z. B. erhält man aus 1-Methylnaphthalin 1-Naphthoesäurenitril und aus 1,8-Diethylnaphthalin 1,8-Naphthalindicarbonsäuredinitril. Man kann auch Alkylaromaten mit längeren Alkylseitenketten, z. B. mit bis 4 Kohlenstoffatomen, verwenden, allerdings wird das Verfahren dann weniger wirtschaftlich.

Die Sauerstoffkonzentration in dem Reaktionsgemisch kann in weiten Grenzen variieren. Vorzugsweise verwendet man einen Sauerstoffüberschuß, etwa das 1,5-fache der theoretisch erforderlichen Menge. An Ammoniak setzt man zweckmäßig einen Überschuß ein, vorteilhaft das 1,2- bis 20-fache der theoretisch erforderlichen Menge.

Die Konzentration des alkylsubstituierten aromatischen Kohlenwasserstoffs in dem Gemisch der Reaktionsteilnehmer beträgt vorzugsweise 1,8 bis 5,0 Volumenprozent, bezogen auf die gesamte gasförmige Reaktionsmischung. Man kann auch eine Verdünnung mit Inertgasen vornehmen, indem man z. B. Luft als Sauerstoff lieferndes Gas verwendet oder indem man das Gasgemisch mit Inertgasen, z. B. mit Stickstoff verdünnt.

Die Umsetzung wird zwischen 300 und 500 °C, vorzugsweise zwischen 420 und 480 °C, durchgeführt. Es empfiehlt sich, besonders bei der Herstellung von Phthalsäuredinitril, Temperaturen zwischen 430 und 480 °C einzuhalten, da mit steigender Reaktionstemperatur der Gehalt an Imid und Diamid abnimmt. Es lassen sich so bei Anwendung von Reaktionstemperaturen von z. B. 450 bis 480 °C Dinitrile mit einer Reinheit von über 99,5 % gewinnen, so daß eine nachfolgende Reinigung und Abtrennung von Imid und Diamid entfallen kann.

Die Verweilzeit des Gasgemisches am Katalysator kann in weiten Grenzen, z. B. zwischen 0,1 und 25 Sekunden varriieren, vorzugsweise beträgt sie etwa 0,5 bis 5 Sekunden.

Man kann mit einem festen Katalysator oder im Fließbettverfahren arbeiten. Zweckmäßig führt man die Umsetzung bei Atmosphärendruck durch. Man kann sie aber auch unter leicht erhöhtem Druck, z. B. bis zu 1,5 atü, oder unter leicht vermindertem Druck, z. B. 300 Torr, ausführen.

Die Durchführung der Reaktion erfolgt in üblicher Weise, z. B. indem man das Gasgemisch bei der Reaktionstemperatur über den Katalysator leitet, dann die Reaktionsgase abkühlt und die Nitrile durch Kondensation, gegebenenfalls durch Einsprühen von Wasser abscheidet. Wegen der großen Reinheit ist in vielen Fällen eine gesonderte Nachreinigung zur Entfernung von gebildetem Imid und Amid nicht erforderlich.

Beispiel 1

In einen Quarzreaktor mit 60 mm Durchmesser und 1 000 mm Länge, der mit Außenheizung und mit einer Quarzfritte zur gleichmäßigen Verteilung des Reaktionsgases versehen ist, werden 750 ml eines Katalysators mit 0,05-0,3 mm Korngröße eingefüllt. Die Zusammensetzung des Katalysators beträgt 5,0 Gew.% $V_2O_5$, 5,9 Gew.% $Sb_2O_3$, 0,24 Gew.% $K_2O$, 0,56 Gew.% $BaO$ und 88,3 Gew.% $\gamma-Al_2O_3$. Der Reaktor wird auf 470 °C erhitzt und ein Gasgemisch aus 3,25 Vol.% o-Xylol, 13 Vol.% Sauerstoff und 83,75 Vol.% Ammoniak hindurchgeleitet. Die heißen Reaktionsgase werden in zwei hintereinander angeordneten Waschtürmen mit Ammoniakwasser auf 70 °C abgekühlt. Es entsteht eine wäßrige Kristallsuspension, aus der das o-Phthalodinitril durch Filtration gewonnen wird. Aus 2 687 Teilen o-Xylol werden 2 581 Teile o-Phthalodinitril und 195 Teile o-Tolunitril erhalten. Durch erneute Reaktion der 195 Teile o-Tolunitril mit Ammoniak und Sauerstoff werden weitere 170 Teile o-Phthalodinitril erhalten. Die Gesamtausbeute an o-Phthalodinitril, bezogen auf umgesetztes o-Xylol beträgt 84,8 Mol.%.

Vergleichsbeispiel

In die gleiche Apparatur werden 750 ml eines erdalkalifreien Vergleichskatalysators mit der Korngröße 0,05-0,3 mm und der Zusammensetzung 5,0 Gew.% $V_2O_5$, 6,8 Gew.% $Sb_2O_3$ und 0,29 Gew.% $K_2O$ auf $\gamma$-Aluminiumoxid eingefüllt. Der Katalysator wird auf 475 °C erhitzt und ein Gasgemisch aus 2,8 Vol.% o-Xylol, 11,7 Vol.% Sauerstoff und 85,5 Vol.% Ammoniak hindurchgeleitet. Die Abscheidung und Aufarbeitung erfolgt wie im Beispiel 1. Aus 2 659 Teilen o-Xylol werden 2 514 Teile o-Phthalodinitril und 240 Teile o-Tolunitril erhalten. Durch erneute Reaktion der 240 Teile o-Tolunitril mit Sauerstoff und Ammoniak werden weitere 197 Teile o-Phthalodinitril erhalten. Die Gesamtausbeute an o-Phthalodinitril beträgt 78,3 Mol.%, bezogen auf eingesetztes o-Xylol.

Beispiel 2

Dem im Beispiel 1 beschriebenen Katalysator wurden anstelle des Bariumoxids 0,5 Gew.% Magnesiumoxid beigemengt und unter gleichen Bedingungen wie im Beispiel 1 die Umsetzung von o-Xylol mit Ammoniak und Sauerstoff durchgeführt.
Aus 3 378 g o-Xylol werden 3 121 g o-Phthalodinitril und 295 g o-Tolunitril erhalten. Durch erneute Umsetzung des o-Tolunitrils werden weitere 262 g o-Phthalodinitril erhalten. Die Gesamtausbeute beträgt 82,9 Mol.%.

Beispiel 3

Anstelle des Bariumoxids werden dem im Beispiel 1 beschriebenen Katalysator 0,15 Gew.% Calciumoxid beigemengt und unter den dort beschriebenen Bedingungen die Reaktion von o-Xylol mit Ammoniak und Sauerstoff durchgeführt.
Aus 2 522 g o-Xylol werden 2 372 g o-Pthalodinitril und 206 g o-Tolunitril erhalten. Durch erneute Umsetzung des o-Tolunitrils werden weitere 180 g o-Phthalodinitril erhalten, was einer Gesamtausbeute von 83,8 Mol.% entspricht.

**Patentanspruch**

Verfahren zur Herstellung von aromatischen Nitrilen, insbesondere von Phthalsäuredinitrilen, aus entsprechend alkylsubstituierten aromatischen Kohlenwasserstoffen durch katalytische Oxydation mit Sauerstoff oder Sauerstoff enthaltenden Gasen in Gegenwart von Ammoniak bei erhöhter Temperatur in der Dampfphase in Gegenwart eines Katalysators, der 2 bis 10 Gew.% Vanadium(V)-oxid, 1 bis 10 Gew.% Antimon(III)-oxid, 0,02 bis 2 Gew.% Alkalioxid und Aluminiumoxid enthält, dadurch gekennzeichnet, daß man dem Katalysator 0,01 bis 1,0 Gew.% eines Erdalkalimetalles, oder einer Verbindung eines Erdalkalimetalles, insbesondere Barium oder einer Bariumverbindung zusetzt.

**Claim**

A process for the preparation of an aromatic nitrile, in particular a phthalodinitrile, from an appropriately alkyl-substituted aromatic hydrocarbon by catalytic oxidation with oxygen or an oxygen-containing gas in the presence of ammonia at elevated temperatures in the vapor phase, in the presence of a catalyst which contains from 2 to 10 % by weight of vanadium(V) oxide, from 1 to 10 % weight of antimony(III) oxide, from 0.02 to 2 % by weight of an alkali metal oxide and alumina, wherein from 0.01 to 1.0 % by weight of an alkaline earth metal, or of a compound of an alkaline earth metal, in particular barium or a barium compound, is added to the catalyst.

**Revendication**

Procédé de préparation de nitriles aromatiques, en particulier de dinitriles de l'acide phtalique, à partir d'hydrocarbures alkyl-substitués de façon appropriée, par une oxydation catalysée à température accrue, dans la phase gazeuse, par de l'oxygène ou un mélange gazeux contenant de l'oxygène, en présence d'ammoniac et d'un catalyseur, qui contient de 2 à 10 % en poids d'oxyde de vanadium pentavalent, de 1 à 10 % en poids d'oxyde d'antimoine trivalent et de 0,02 à 2 % en poids d'oxyde de métal alcalin et d'oxyde d'aluminium, caractérisé en ce que l'on incorpore au catalyseur 0,01 à 1,0 % en poids d'un métal alcalino-terreux ou d'un dérivé d'un métal alcalino-terreux, en particulier de baryum ou d'un dérivé du baryum.